# EUROPEAN PATENT APPLICATION

(11) **EP 2 591 810 A1**
(43) Date of publication of application: **15.05.2013**
(21) Application number: 12000377.7
(22) Date of filing: 20.01.2012
(51) Int. Cl.: A61L 9/22, F24F 3/16, H01T 23/00, B60H 3/00

(54) **Wind power negative ion generator**

(30) Priority: 11.11.2011 TW 00221406
(71) Applicant: Lai, Dah Prong, Bali Distr New Taipei City 249 (TW)
(72) Inventor: Lai, Dah Prong, Bali Distr New Taipei City 249 (TW)
(74) Representative: Zeitler - Volpert - Kandlbinder

(57) **Abstract**

A wind power negative ion generator includes a negative ion generator main body and a wind power generation device coupled to the negative ion generator main body. The wind power generation device has a power cord connected to a power line of the negative ion generator to supply electricity. The wind power generation device includes fan blades to communicate with outside and form a wind receiving side. The negative ion generator of the present invention can be widely used, not limited to a fixed power source socket. The negative ions generated by the present invention can move along with the wind to enlarge the range of the negative ions.

## Description

### BACKGROUND OF THE INVENTION

### 1. Field of the Invention

The present invention relates to a wind power negative ion generator, and more particularly to a negative ion generator can be moved and widely used at different places and locations, not limited to a fixed power source socket.

### 2. Description of the Prior Art

When water changes its surface in the air, for example a water drop becomes more small water drops by fission, the surrounding air will get negative charge to generate negative ions which can make people comfortable. The negative ions will combine with the dust in the air to drop by static electricity so as to purify the air. The negative ions are beneficial to human health. The working principle of a conventional negative ion generator is that 3-7KV DC voltage is applied to a metallic needle or carbon fibers (negative ion emitting end), so that the surrounding air of the emitting end is ionized to negative ions. The dust and smoke in the air are combined with the negative ions to drop by static electricity so as to purify the air.

The negative ion generator with carbon fiber negative ion emitting end comprises a power line to connect with an external power source. In general, the exposed emitting end is the end of a high tension line. The high tension line is wrapped by an insulating sleeve, with the terminal to connect with a partial exposed soft carbon brush emitter. After the power line is connected with the external power source, the terminal of the high tension line with the soft carbon brush emitter generates negative ions to purify the surrounding air.

The conventional negative ion generator is widely used to various vehicles or indoor and food preservation places. The main shortcoming of the conventional negative ion generator is to find the corresponding voltage and the suitable power socket (such as, a household socket, a vehicular cigarette lighter or an indoor socket). The conventional negative ion generator cannot be moved, so its use is limited.

### SUMMARY OF THE INVENTION

The primary object of the present invention is to provide a wind power negative ion generator. The negative ion generator can be widely used, not limited to a fixed power source socket.

In order to achieve the aforesaid object, the wind power negative ion generator of the present invention comprises a negative ion generator main body and a wind power generation device coupled to the negative ion generator main body. The wind power generation device has a power cord connected to a power line of the negative ion generator to supply electricity. The wind power generation device comprises fan blades to communicate with outside and form a wind receiving side.

Preferably, the wind power generation device further has an opposing wind sending side relative to the wind receiving side.

Preferably, the negative ion generator main body further comprises a positioning device. The positioning device is one of a clip member, a Velcro, a fastening strip and a twin adhesive.

Preferably, the negative ion emitting end is a soft carbon brush or a metallic needle.

Preferably, the negative ion generator main body further comprises a voltage regulation device to cooperate with the rotational speed of the fan blades of the wind power generation device.

Preferably, the wind power generation device further comprises a wind speed throttle to cooperate with different rotational speeds of the fan blades of the wind power generation device.

Preferably, the fan blades of the wind power generation device are mounted in a wind guiding room.

Preferably, the negative ion emitting end faces the wind sending side to generate a convection effect when in use for dissipating negative ions evenly and enlarging the range of the negative ions.

### BRIEF DESCRIPTION OF THE DRAWINGS

Fig. 1 is a perspective view according to a preferred embodiment of the present invention;
Fig. 2 is a rear view of Fig. 1;
Fig. 3 is a left side view of Fig. 2;
Fig. 4 is a perspective view of the wind speed throttle of the present invention; and
Fig. 5 is another perspective view of Fig. 4.

### DETAILED DESCRIPTION OF THE PREFERRED EMBODIMENTS

Embodiments of the present invention will now be described, by way of example only, with reference to the accompanying drawings.

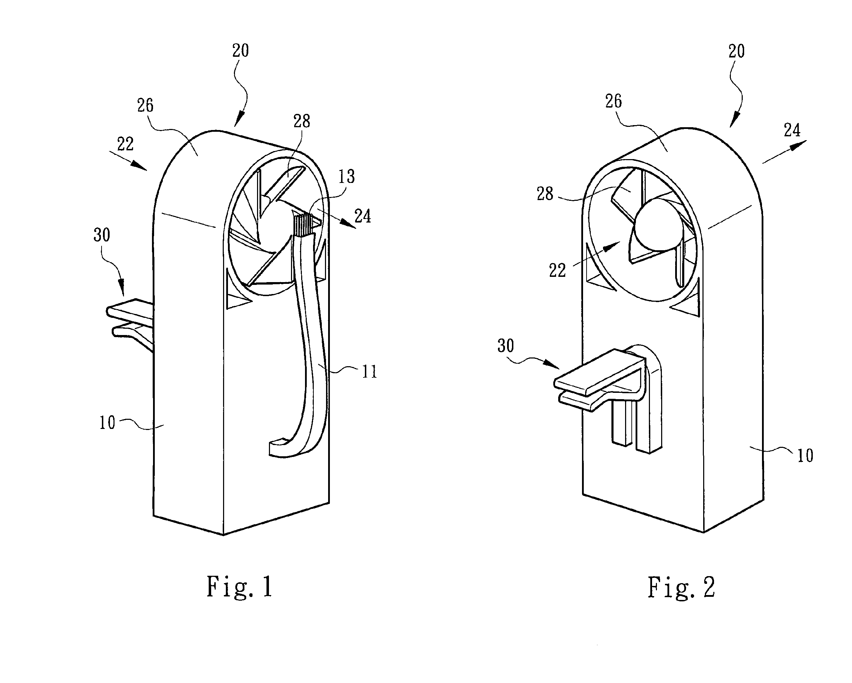

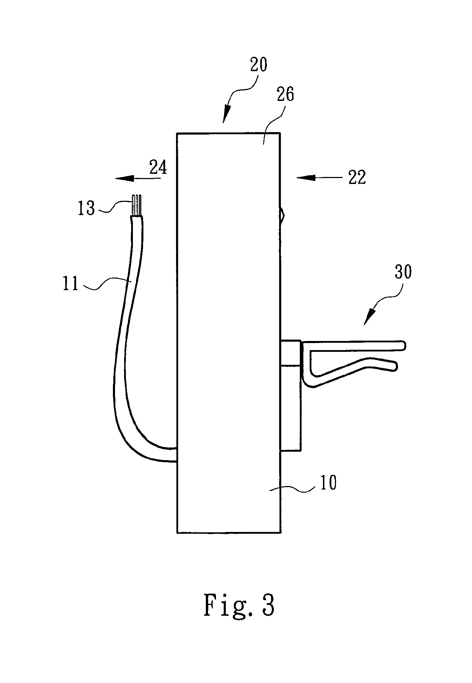

As shown in Fig. 1 through Fig. 3, the present invention comprises a negative ion generator main body 10 to accommodate the negative ion generator. The generative ion generator has a flexible high tension line 11 with a soft carbon brush negative ion emitting end 13. The high tension line 11 and the negative ion emitting end 13 are exposed out of the negative ion generator main body 10. The soft carbon brush negative ion emitting end 13 is just as an example and not limited, which can be other type of negative ion emitting end, such as a metallic needle.

A wind power generation device 20 is coupled to the negative ion generator main body 10. The power cord of the wind power generation device 20 is connected to the negative ion generator. In general, the exposed power cord supplies power to the negative ion generator. The wind power generation device 20 comprises fan blades 28 which communicate with outside and are turned by the wind power of an external wind source including other power device or fan device to generate the required electricity.

As shown in the drawings, the fan blades 28 of the wind power generation device 20 are disposed at one side as indicated by the arrow to form a wind receiving side 22 (facing the wind outlet of the wind power source, other power device or fan device). The opposing side as indicated by another arrow is a wind sending side 24.

In this embodiment, the fan blades 28 of the wind power generation device 20 are mounted in a wind guiding room 26 to guide the wind power from the wind receiving side 22 to the wind sending side 24.

In this embodiment as shown in the drawings, the negative ion emitting end 13 preferably faces the wind sending side 24 as indicated by the arrow to enhance the diffusion of the negative ions of the device.

The aforesaid embodiment of the present invention further comprises a positioning device 30 to position the negative ion generator main body 10 at a desired article or a place. In this embodiment as shown in the drawings, the positioning device 30 is a clip member coupled to the negative ion generator main body 10, such that the entire device can be positioned at the wind outlet of an indoor or vehicular electric fan or air conditioner.

The aforesaid positioning device is just as an example, which can be a Velcro, a fastening strip, or a twin adhesive or the like to position the present invention at a desired place.

Furthermore, the present invention further comprises a voltage regulation (such as, boosting voltage or stabilizing voltage) device for the fan blades when they are turned slowly or for the related circuit to supply power to the negative ion generator.

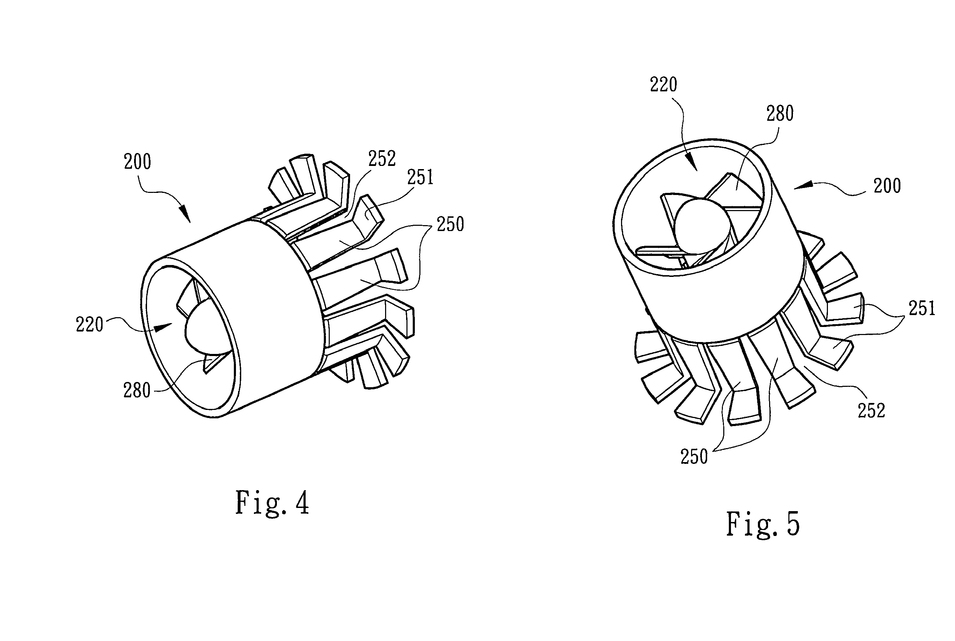

Furthermore, the present invention further comprises a wind speed throttle structure for the wind blades to have even power generation when at different rotational speed (high speed or lower speed). As shown in Fig. 4 and Fig. 5, the wind power generation device 200 of the present invention comprises the wind speed throttle structure. The opposing side of the wind receiving side 220 of the fan blades 280 is provided with a plurality of spaced resilient claws 250 which are arranged in a circle. Each resilient claw 250 has a protrusion 251. A gap 252 is defined between every two adjacent resilient claws 250. When the fan blades 280 are turned at different speeds, the opposing side of the wind receiving side 220 forms different wind resistance structures to achieve the wind speed throttling effect.

The present invention is portable and movable to be installed at different places, such as the cool air outlet of the household or office air conditioner. The negative ion emitting end is adjusted to the required wind power flow direction. The fan blades 28 of the wind power generation device 20 are driven by the wind power to supply electricity to the negative ion generator for the emitting end 13 to generate negative ions. The present invention can be installed on mobile vehicles, for example, it is installed at the cool air outlet of an automobile. The present invention can be positioned on the exterior of a helmet to generate negative ions by movement of the motor vehicle so as to purify the air.

Although particular embodiments of the present invention have been described in detail for purposes of illustration, various modifications and enhancements may be made without departing from the spirit and scope of the present invention. Accordingly, the present invention is not to be limited except as by the appended claims.

## Claims

1. A wind power negative ion generator, comprising a negative ion generator main body, the generative ion generator comprising a power line and an exposed negative ion emitting end disposed at an end of a high tension line; at least one wind power generation device coupled to the negative ion generator main body, the wind power generation device having a power cord connected to the power line of the negative ion generator; the wind power generation device comprising fan blades to communicate with outside and form a wind receiving side.

2. The wind power negative ion generator as claimed in claim 1, wherein the wind power generation device further has an opposing wind sending side relative to the wind receiving side.

3. The wind power negative ion generator as claimed in claim 1 or claim 2, wherein the negative ion generator main body further comprises a positioning device.

4. The wind power negative ion generator as claimed in claim 3, wherein the positioning device is one of a clip member, a Velcro, a fastening strip and a twin adhesive.

5. The wind power negative ion generator as claimed in any claims of claim 1 to claims 4, wherein the negative ion emitting end is a soft carbon brush.

6. The wind power negative ion generator as claimed in any claims of claim 1 to claims 4, wherein the negative ion emitting end is a metallic needle.

7. The wind power negative ion generator as claimed in any claims of claim 1 to claims 4, wherein the negative ion generator main body further comprises a voltage regulation device.

8. The wind power negative ion generator as claimed in any claims of claim 1 to claims 4, wherein the wind power generation device further comprises a wind speed throttle.

9. The wind power negative ion generator as claimed in any claims of claim 1 to claims 4, wherein the fan blades of the wind power generation device are mounted in a wind guiding room.

10. The wind power negative ion generator as claimed in any claims of claim 1 to claims 4, wherein the negative ion emitting end faces the wind sending side.
